# EUROPEAN PATENT SPECIFICATION

(11) **EP 3 355 943 B1**
(45) Date of publication and mention of the grant of the patent: **03.06.2020**
(21) Application number: 16784604.7
(22) Date of filing: 20.09.2016
(51) Int. Cl.: A61L 2/22, A61K 31/20

(54) **METHODS AND SYSTEMS FOR TREATING MEDICAL DEVICES AND FLUIDS**
VERFAHREN UND SYSTEME ZUR BEHANDLUNG MEDIZINISCHER GERÄTEN UND FLÜSSIGKEITEN
MÉTHODES ET SYSTÈMES POUR LE TRAITEMENT DE FLUIDES ET DE DISPOSITIFS MÉDICAUX

(30) Priority: 02.10.2015 US 201562236632 P
(43) Date of publication of application: 08.08.2018
(73) Proprietor: Ethicon, Inc., Somerville, NJ 08876 (US)
(72) Inventor: VAN HOLTEN, Robert W., Somerville, New Jersey 08876 (US)
(74) Representative: Kirsch, Susan Edith
(86) International application number: PCT/US2016/052584
(87) International publication number: WO 2017/058563

(56) References cited:
- WO-A1-91/16410
- WO-A2-03/009704
- US-A- 4 808 314
- US-A1- 2011 230 558

## Description

### FIELD OF THE INVENTION

The present invention relates to methods and systems for reducing lipopolysaccharides an medical devices and fluids that are intended to be in contact with the human body or to be inside of the human body.

### BACKGROUND OF THE INVENTION

Lipopolysaccharides (LPS), also known ae lipoglycans and endotoxin, are large molecules consisting of a lipid and a polysaccharide. LPS is the major component of the outer membrane of gram-negative bacteria, contributing to the structural integrity of the bacteria, and protecting the membrane from certain kinds of chemical attack. It is of crucial importance to gram-negative bacteria, whose death results if it is mutated or removed.

LPS induces a strong response from normal animal immune systems, such as human immune system. The presence of endotoxins in the blood is called endotoxemia. It can lead to septic shock, if the immune response is severely pronounced. The levels of endotoxin allowed on medical devices that have circulatory, neural, and ophthalmic exposure are tightly regulated.

Some commonly und techniques for removing endotoxin contaminants are ultrafiltration and ion exchange chromatography. Ultrafiltration, although effective in removing endotoxins from water, is an inefficient method in the presence of proteins, which can be damaged by physical forces. Anion exchangers, which take advantage of the negative net charge of endotoxins, have been extensively used for endotoxin adsorption. However, when negatively charged proteins need to be decontaminated, they may co-adsorb onto the matrix and cause a significant loss of biological material. Also, not-positively charged proteins can form complexes with endotoxins. Prior removal attempts have included numerous approaches which require costly equipment, laborious processes, and limited in scope being applied to labile and costly pharmaceuticals and biologies.

In the pharmaceutical industry several alternative routes are known to generate products with low-endotoxin levels. However, their diversity indicates a dilemma in endotoxin removal. Several procedures were developed for pharmacoproteins, taking advantage of the characteristics of the production process, tailored to suit specific product requirements. Therefore, each procedure addresses the problem in a completely different way; none of them turns out to be broadly applicable. Anionic-exchange chromatography, for example, is potentially useful for the decontamination of positively-charged proteins, such as urokinase. However, decontamination of negatively-charged proteins would be accompanied by a substantial loss of the product due to adsorption. For small proteins, such as myoglobin (18000 Da), ultrafiltration can be useful to remove large endotoxin aggregates. With large proteins, such as immunoglobulins (150000 Da) ultrafiltration would not be effective. In addition, ultrafiltration would fail if interactions between endotoxins and proteins cause endotoxin monomers to permeate with proteins through the membrane. Currently, there has not been a universal means to remove endotoxin for pharmaceutical and biological applications which has resulted in a milieu of procedures custom designed to the specific product.

Endotoxins can be considered to be temperature and pH stable, rendering their removal as one of the most difficult tasks in downstream processes during protein purification. Two important factors influencing the success of any approach are the affinity of the endotoxin and protein antigen for the chromatography support or media used and the affinity of the endotoxin for the protein antigen. A third factor is how the affinity of the endotoxin for the protein can be modified by factors such as temperature, pH, detergents (surfactants), solvents and denaturants.

Usually, the procedures employed for endotoxin removal are unsatisfactory regarding selectivity, adsorption capacity and recovery of the protein. In the selective removal of endotoxin from protein-free solutions, it is easy to remove endotoxins by ultrafiltration taking advantage of the different sizes of the endotoxin and water, or by non-selective adsorption with hydrophobic adsorbent or an anion-exchanger.

Some commonly used techniques for removing endotoxin contaminants are ultrafiltration and ion exchange chromatography. Ultrafiltration, although effective in removing endotoxins from water, is an inefficient method in the presence of proteins, which can be damaged by physical forces. Anion exchangers, which take advantage of the negative net charge of endotoxins, have been extensively used for endotoxin adsorption. However, when negatively charged proteins need to be decontaminated, they may co-adsorb onto the matrix and cause a significant loss of biological material. Also, net-positively charged proteins form complexes with endotoxins, causing the proteins to drag endotoxin along the column and consequently minimizing the endotoxin removal efficiency.

In order to remove endotoxin from recombinant protein preparations, the protein solution may be passed through a column that contains polymyxin B immobilized on Sepharose 4B, in the hope that contaminating endotoxin binds to the gel. Similarly, histidine immobilized on Sepharose 4B also has the capability to capture endotoxin from protein solutions. Polymyxin B affinity chromatography is effective in reducing endotoxin in solutions. Polymyxin B, a peptide antibiotic, has a very high binding affinity for the lipid A moiety of most endotoxins. Karplus et al. in an article titled "A new method for reduction of endotoxin contamination from protein solutions", J. Immunol. Methods 1987 Dec 24; 105(2): 211-20, reported an improved method of polymyxin B affinity chromatography in which endotoxin could be absorbed effectively after dissociation of the endotoxin from the proteins by a nonionic detergent, octyl-β-D-glucopyranoside.

An article titled "Endotoxin removal devices for the treatment of sepsis and septic shock" by B Davies, J Cohen, Lancet Infect Dis 2011; 11: 65-71 describes polymyxins, a group of cyclic cationic polypeptide antibiotics. In addition to their antimicrobial property, polymyxins can bind to and neutralize endotoxin. The article discusses the possibility of using polymyxin bound to a solid-phase carrier for specific haemadsorption in patients with sepsis, thereby retaining the lipopolysaccharide-binding properties but minimizing systemic toxic effects. This system has been widely used in Japan for many years, but convincing clinical evidence of efficacy is lacking. A recent Italian study has some promising data. Although polymyxin has been the principal agent used to explore this approach, other molecules have the ability to bind endotoxin, and some of these have very recently been proposed as the basis for other endotoxin-removal devices. The available evidence is reviewed to assess the potential use of such devices in clinical practice.

The methods mentioned above are reasonably effective for removal of endotoxins from protein solutions with relatively high protein recoveries. However, these affinity phases cannot be cleaned with standard depyrogenation conditions of strong sodium hydroxide in ethanol. These supports suffer from considerable efficiency decrease in the presence of proteins. Hence, they are not in general applicable for the above mentioned problem.

Membrane-based chromatography has been successfully employed for preparative separations; predominantly for protein separations. Nevertheless, universal adoption of this technology has not taken place because membrane chromatography is limited by the binding capacity.

Calcium stearate is used in numerous medical applications, including such products as sutures, pharmaceuticals, and catheters. The pharmaceutical and cosmetics industry often use calcium stearate as an anti-caking additive for powders and granules and as an excipient for pressing tablets. Salts of stearic acid are "generally recognized as safe" (GRAS) substances and are found in animals and are used widely as excipients.

U.S. Patent No. 4,185,637 "Coating composition for sutures " discloses a multifilament suture having improved tie-down properties, said suture being coated with from about 1 to 5 percent by weight of the dry residue of a composition comprising a gel of a polyvalent metal ion salt of a C₆ or higher fatty acid in a volatile organic solvent. The fatty acid salt can be salt of calcium, magnesium, barium, aluminum, or zinc. The fatty acid salt can further be the salt of calcium or magnesium, or the fatty acid salt can be calcium stearate.

Published PCT application WO 2013/092416 "Drug-coated medical devices" discloses medical devices carrying at least on a portion of its surface at least one drug and at least one lipophilic lubricant at a ratio of 0.1 -500% by weight of the at least one lipophilic lubricant in relation to 100% by weight of the drug, wherein the at least one drug is selected from paclitaxel, arsenic trioxide, lipophilic derivatives of corticoids and Sirolimus, everolimus, zotarolimus, biolimus, temsirolimus and the at least one lipophilic lubricant is a C₆-C₃₀-monocarboxylic acid salt and the at least one drug and the at least one lubricant are applied at the same time in the same solvent or mixture of solvents or the drug-coated device is coated with an additional layer of the at least one lubricant. It further discloses medical device wherein the C₆-C₃₀- monocarboxylic acid salt is selected from magnesium stearate, calcium stearate, zinc stearate, magnesium palmitate, calcium palmitate, zinc palmitate, magnesium myristate, calcium myristate, magnesium laurate, calcium laurate, magnesium caprinate, calcium caprinate, magnesium caprylate, calcium caprylate, magnesium oleate, calcium oleate, magnesium palmitoleate or calcium palmitoleate.

WO 03/009704 A2 discusses an enteral composition containing phospholipids, triglycerides and cholesterol or precursors thereof, which can be used in the treatment of sepsis.

US 4 808 314 A discusses a method of reducing a bacterial endotoxin contaminant in a biologically useful macromolecule. An aqueous medium containing an endotoxin-contaminated macromolecule is admixed with a dialyzable surfactant, and the admixture so formed is contacted with an endotoxin sorbant to form a solid-liquid phase admixture. The contacting is maintained until the endotoxin is bound to the sorbant. The surfactant is dialyzed out of the aqueous liquid phase at a time no earlier than the maintenance step. The liquid phase containing the macromolecule is separated and recovered.

US 2011/230558 A1 discusses a use of a composition for the inactivation of toxins containing cationic surfactants such as ethyl-N^{α}-lauroyl-L-arginate HCI (LAE). It has been found that cationic surfactants such as ethyl-N^{α}-lauroyl-L-arginate HCI (LAE) and its salts are effective against endotoxins, exotoxins and aflatoxins.

WO 91/16410 A1 discusses a method of precision cleaning a medical device by contacting it with a chlorine-containing cleansing agent so as to remove pyrogens from the medical device.

There is a need to replace other techniques or LPS removal such as nanofiltration or charged filtration which are complex, expensive, or may not work with certain components such as biologics.

### SUMMARY OF THE INVENTION

Briefly, the present invention is directed to methods and systems for reducing lipopolysaccharides on medical devices and fluids that are intended to be in contact with the human body or to be inside of the human body. In one embodiment, the present invention relates to methods for treating solutions containing one or more endotoxins at detectable levels by adding a suspension of a salt of stearic acid, preferably calcium stearate, to said solution to reduce detectable amounts of said endotoxins; and optionally removing the salt of stearic acid. The solution is aqueous or more preferably, water, or physiological saline.

In an alternative embodiment, the present invention is directed to methods for treating medical devices by contacting the devices with a suspension of a salt of stearic acid in water, saline or in non-aqueous solvents to reduce detectable amounts of endotoxins on said medical device. The device can, for example, be a suture. The device is preferably contacted with water or saline and the salt of stearic acid is calcium stearate.

In an alternative embodiment, the present invention is directed to a suspension of a salt of stearic acid for use in a method of reducing detectable amounts of endotoxins on a mammal, wherein said method comprises contacting the tissue surface of a mammal with a suspension of a salt of stearic acid. The suspension of a salt of stearic acid may be calcium stearate. The suspension of a salt of stearic acid may be delivered in combination with antibiotic therapy. The salt of stearic acid suspension may be delivered into/onto the gastrointestinal tract of a mammal, and the suspension of a salt of stearic acid may be delivered in a quantity at least equal to the quantity of antibiotic. The suspension of a salt of stearic acid may contain calcium stearate suspended in a water/saline carrier and this may be used as a lavage to remove endotoxin from injury sites prior to surgery or to reduce the severity and/or occurrence of sepsis.

### DETAILED DESCRIPTION

The inventors discovered that when certain salts of stearic acid, such as calcium stearate, are placed in direct contact with systems, devices, or fluids (such as water), containing LPS, the endotoxin activity can be controlled. Calcium stearate, generally thought of as a salt, is a compound comprising of two long-chain fatty acids with calcium and is substantially insoluble in water at room temperature. These straight-chained saturated, monobasic acids are found abundantly in animal fats and in varying degrees in cottonseed, corn, soya, coco and palm oils. In their pure state, these acids are solid crystalline, opaque white materials having a waxy feel. It has multiple applications in different production processes varying from lubricants to food and medical drugs in the form of a stabilizer or emulsifier.

According to one aspect of the present invention, liquids, such as water or aqueous solutions, including any biologics (e.g. proteins) solutions or suspensions, purification is performed using salts of stearic acid, such as calcium stearate (CaSt). The effect on detectable LPS was dependent on the dose of the salt of stearic acid, temperature independent, and showed rapid onset of the LPS control effect. Without being bound by any theory, it appears that salts of stearic acid such as CaSt inactivate LPS so that it is not even detectable by the standard assay. A particularly preferred quantity of CaSt is 0.2g CaSt/5mL of liquid. However, it has been shown to reduce LPS in the examples below even at a lower concentration of 0.007g CaSt/5mL of liquid.

In one aspect, CaSt powder suspension is added to the treated liquid, with optional agitation. After an exposure time ranging from several seconds to several hours, the liquid is substantially free of measurable LPS. Optionally, the liquid can be separated from the salt of stearic acid powder by any known method, such as filtration, decanting, precipitation, centrifugation, etc., or combinations thereof, as a result of the lack of aqueous solubility of the salt of stearic acid.

In another aspect, aqueous-containing liquids can be treated by passing through or over salts of stearic acid which are supported or immobilized on a column or on a filter. In yet another aspect, such liquids can be treated by passing through a fluidized or packed bed filled with salt of stearic acid particles.

According to one aspect of the present invention, medical devices, such as sutures, needles, meshes, syringes, implants, contact lenses, medical devices storage containers, etc., are treated in an aqueous suspension containing salts of stearic acid such as CaSt, with optional agitation. Exposing medical devices to a suspension of CaSt in water is expected to eliminate LPS from the surface of the medical device. In one aspect, CaSt powder is added to water, forming a suspension, and the treated medical device is immersed in the suspension, with optional agitation.

It is not always desirable to expose medical devices, particularly absorbable medical devices, to water or aqueous solutions. According to one aspect of the present invention, medical devices, particularly absorbable medical devices, such as sutures, meshes, etc., are treated in a solvent containing a suspension of a salt of stearic acid or solution, optionally with agitation. In one aspect, CaSt powder is added to an organic solvent-containing solution, and the treated medical device is immersed in the solvent, with optional agitation. After an exposure time ranging from several seconds to several hours, the device is removed and optionally dried or washed. Examples of suitable organic solvents include acetates, such as alkyl acetate,

particularly ethyl acetate, alcohols, such as C₁-C₆ alcohols, particularly lower alkyl alcohols, such as propanol, isopropanol and butanol, and cyclic alkanes, such as cyclohexane.

According to one aspect of the present invention, biological solutions can be treated with suspensions of salts of stearic acid. In one aspect, CaSt powder suspension can be added to the treated liquid, with optional agitation. After an exposure time ranging from several seconds to several hours, the liquid is free of LPS and can be used. Optionally, the liquid can be separated from salt of stearic acid powder by any known method, such as filtration, decanting, precipitation, centrifugation, etc., or combinations thereof.

In another aspect, liquids can be treated to eliminate LPS by passing through or over salts of stearic acid which are supported or immobilized on a column or on a filter. In yet another aspect, liquids can be treated by passing through a fluidized or packed bed filled with salt of stearic acid particles.

In another aspect blood or blood products or derivatives can be treated to eliminate LPS by passing through or over salts of stearic acid which are supported or immobilized on a column or on a filter, or can be stored exposed to salts of stearic acid as suspensions, powders, or coatings on storage vessels. According to this aspect, storage time of blood and blood products can be increased. Effects of LPS on platelet function is expected to be negated by salt of stearic acid, such as CaSt. Currently, platelet concentrates are held at room temperature for up to five days prior to administration. Any gram negative bacteria introduced to the platelets during collection will continue to proliferate during storage. This contamination can result in platelets being disposed of and also mild to severe reaction post administration of this life saving blood product. By exposing platelets to calcium stearate or equivalent compounds, cost and efficacy will be significantly improved.

A method for treating for LPS related conditions, either prophylactically or for acute conditions, in mammals, including humans and animals is provided. The treatment can be contemplated by treating blood, or treating the GI tract with salts of stearic acid in various forms, including suspensions and immobilized salts of stearic acid. Oral dose forms containing salts of stearic acid in large quantities are contemplated. Salts of stearic acid as additives to oral or topical dose forms such as antibiotics are contemplated. Any form of salts of stearic acid for administration can be utilized, including solid dose forms, powders, suspensions, and similar.

In one aspect, salts of stearicacid are supplied prior to administration of antimicrobial agents e.g. antibiotics. In one aspect, salts of stearicacid are supplied after administration of antimicrobial agents e.g. antibiotics. In one aspect, salts of stearic acid are supplied simultaneously with administration of antimicrobial agents e.g. antibiotics. In one aspect, salts of stearic acid are supplied in any combination of administering prior to, simultaneously with, or after administration of antimicrobial agents e.g. antibiotics.

In another aspect, a surgical wash can be applied to any part of the mammalian body, wound, or tissue which is within the body, attached to the body, or is separated from the body, the wash comprising a suspension of a salt of stearic acid such as CaSt. The surgical wash can be applied before, during, or after a surgical procedure, or in any combination of before, during, or after surgical procedure.

In another aspect, the the initiation of sepsis may be reduced through the use of salts of stearic acid or CaSt in suspension as a lavage. When this material is used in such a manner it is placed in the affected area and then removed through aspiration. Any endotoxin that is bound to cells is removed along with the endotoxin that is neutralized by CaSt in the lavage solution. In this way free endotoxins, protein bound endotoxins, and CaSt bound endotoxins are removed, significantly reducing the body's interaction and mitigating any physiological effect.

A method of selective decontamination of the intraperitoneal cavity is provided. Reduction of the Gram negative bacterial load [such as in in the intraperitoneal cavity] would be followed by a decrease in sepsis and bacteremia. This could be accomplished by applying the CaSt into a deliverable surgical wash form. Minimal concentrations of CaSt are required to be efficacious and its mechanism of action will rapidly neutralize endotoxin.

In one aspect, Calcium Stearate is introduced directly into the circulatory system. In another aspect, Calcium Stearate is introduced indirectly as would be required with extracorporeal removal of endotoxin.

In another aspect the salt of stearic acid, such as CaSt, is micronized to form particulates having dimensions from 1 nm to 1 mm, such as 5nm to 500 µm, such as about 10 nm, 100 nm, 1 µm, 10 µm, 50 µm, 100 µm, 300 µm, 500 µm, or combinations thereof.

### EXAMPLE 1.

Water and aqueous solutions (containing LPS with various sources of endotoxin) were exposed to calcium stearate [CaSt] (as a powder suspension) and showed decreased LPS or alternatively do not show any detectable LPS (the effect was CaSt dose dependent). Calcium Stearate powder (PMC BIOGENIX, <21.8 microns, vegetable based) in various quantities was added to sterile test tubes. 5 mL of water solution of known endotoxin concentration (generated by spiking endotoxin-free water with water solution of known endotoxin concentration) was added to the each test tube, vortexed vigorously (1 minute) using Fisher Scientific Digital Vortex Mixer, and then placed in a warm water bath (37°C) for 1 hour. After 1 hour, the tubes were removed from warm water bath and vortexed vigorously (1 minute). Sterile syringes with needles were used to extract the solution without removing the calcium stearate. This solution was then tested for endotoxin levels. Kinetic Chromogenic Method for Endotoxin Quantitation, using Endosafe MCS Multi Cartridge System by Charles River Laboratories, Charleston S.C.

Table 1 shows the results of a test whereby CaSt was added to 5 mL test tubes which contained 5 ml of water spiked with 200 µL of solution containing LPS. The total LPS added to test tubes was about 5 EU, with the detected LPS after the treatment was 3% to 8% of the initial concentration. The data indicates that the amount of detectable LPS decreased significantly upon addition of about 0.25 g of CaSt to 5 ml of water.

**Table 1. Detection of LPS after adding CaSt to test tubes containing water spiked with LPS**

| Sample | Weight of CaSt powder added (g) | Concentration of Spike (EU/mL) | Volume of Spike (µL) | Endotoxin Spiked (EU) | Endotoxin Detected (EU) | Percent of detected Endotoxin vs. initial concentration |
|---|---|---|---|---|---|---|
| 1 | 0.252 | 26.04 | 200 | 5.208 | <0.15 | <2.88% |
| 2 | 0.258 | 26.04 | 200 | 5.208 | <0.42 | <8.06% |
| 3 | 0.272 | 26.04 | 200 | 5.208 | <0.15 | <2.88% |
| 4 | 0.267 | 26.04 | 200 | 5.208 | <0.18 | <3.46% |

Table 2 shows the results of a similar test whereby CaSt was added to 5 mL test tubes which contained 5 ml of water spiked with 1000 µL of solution containing LPS, when LPS concentration was much higher. The total LPS added to test tubes was about 14400 EU, with the detected LPS after the treatment was 0.07%-0.08% of the initial concentration. The data indicates that the amount of detectable LPS decreased significantly upon addition of about 0.25 g of CaSt to 5 ml of water.

**Table 2. Detection of LPS after adding CaSt to test tubes containing water spiked with LPS**

| Sample | Weight of CaSt powder added (g) | Concentration of Spike (EU/mL) | Volume of Spike (µL) | Endotoxin Spiked (EU) | Endotoxin Detected (EU) | Percent of detected Endotoxin vs. initial concentration |
|---|---|---|---|---|---|---|
| 1 | 0.254 | 14,400 | 1,000 | 14,400 | 12.1 | 0.08% |
| 2 | 0.2607 | 14,400 | 1,000 | 14,400 | 10.3 | 0.07% |

Table 3 shows the results of a similar test whereby CaSt was added to 5 mL test tubes which contained 5 ml of water spiked with 1000 µL of solution containing LPS, when LPS concentration was much higher. The total LPS added to test tubes was about 14400 EU, with the detected LPS after the treatment was CaSt dependent and was a function of the amount of CaSt added. CaSt addition ranged about 100 fold from 0.0027 g to 0.25 g, with the detected LPS after the treatment was ranging from 77% to 0.19% of respectively as a percent of the initial concentration. The data indicates that the amount of detectable LPS decreased to below 1% of the initial concentration upon addition of about 0.05-0.25 g of CaSt to 5 ml of water, more specifically to below 0.2% of the initial concentration. At lower quantities of CaSt, LPS decreased not as significantly, with addition of 0.01 g of CaSt resulting in detection of 20% of LPS after the treatment, and addition of 0.0027 g of CaSt resulting in detection of 77% of LPS after the treatment. The observed concentration response indicates that with an increase in calcium stearate mass, the detected endotoxin activity approaches zero. In summary, there are three factors that drive the salts of stearic acid performance to remove/neutralize endotoxins. These three factors, not necessarily listed in order of importance, are concentration, time of exposure, and energy in solution at the micro level.

**Table 3. Detection of LPS after adding CaSt to test tubes containing water spiked with LPS**

| Sample | Weight of CaSt powder added (g) | Concentratio n of Spike (EU/mL) | Volume of Spike (µL) | Endotoxin Spiked (EU) | Endotoxin Detected (EU) | Percent of detected Endotoxin vs. initial concentration |
|---|---|---|---|---|---|---|
| 1 | 0.2502 | 14,400 | 1,000 | 14,400 | 26.7 | 0.19% |
| 2 | 0.0518 | 14,400 | 1,000 | 14,400 | 9.4 | 0.07% |
| 3 | 0.0108 | 14,400 | 1,000 | 14,400 | 2,925 | 20.31% |
| 4 | 0.0027 | 14,400 | 1,000 | 14,400 | 11,055 | 76.77% |

The data shows that water treatment with CaSt results in significantly decreased LPS or endotoxin activity detectable, with the LPS level approaching zero at higher quantities of CaStadded.

### EXAMPLE 2. SUTURE TESTING IN AQUEOUS SUSPENSION

Treatment of sutures in aqueous suspensions of CaSt was performed as follows: Suture used was VICRYL® (Polyglactin 910) suture made of a copolymer made from 90% glycolide and 10% L-lactide, Size 1, 27 inch pieces. Endotoxin sources used were sitting (or stagnant) Rain Water (SRW) and diluted with LRW to obtain a concentration of (3980 EU/mL). Endosafe LAL Reagent Water (LRW) was used, manufactured by Charles River Laboratories, Charleston S.C.. Calcium Stearate used was PMC BIOGENIX, <21.8 microns, Vegetable based).

Eight strands of suture were placed in tubing with LPS (endotoxin) solution for 10 minutes at a time. The sutures were dried for 20 minutes and then placed in round bottom tubes. Suspensions of 5% by weight CaSt in LAL Reagent Water were prepared. (0.5 g CaSt in 10 mL LRW). One suture was placed in each tube and vortexed in this solution for approximately 10 seconds. The sutures were removed and allowed to dry for 20 minutes and then placed in round bottom tubes. This was done for 3 sutures. Three pieces of suture, serving as the control, were not submerged in any solutions and placed in test tubes. 3 mL of LAL reagent water was added to each test tube, vortexed vigorously for 1 minute, and then placed in a hot water bath at 37°C for 1 hour. They were then removed, vortexed vigorously for 1 minute, diluted, and tested on the MCS system. The water that was mixed with the calcium stearate was also tested. Two sutures were submerged in LRW that was mixed with calcium stearate. The LRW was separated from the calcium stearate before two sutures were submerged. These were also tested for endotoxin on the MCS. The results are presented in Tables 4-6.

**Table 4. Detection of LPS after adding CaSt to test tubes containing water spiked with LPS**

| Suture Sample | Solution Submerged In | Endotoxin Detected (EU) | Average Endotoxin Detected (EU) | Average Change in Endotoxin Level (%) |
|---|---|---|---|---|
| 1 | None | 74.4 | 84 | n/a |
| 2 | None | 99 | | |
| 3 | None | 78.6 | | |
| | | | | |
| 4 | LAL Water w/ 5% Calcium Stearate | 5.16 | 3.27 | -96.1% |
| 5 | LAL Water w/ 5% Calcium Stearate | 1.89 | | |
| 6 | LAL Water w/ 5% Calcium Stearate | 2.76 | | |
| | | | | |
| 7 | Water after Calcium Stearate | 13.56 | 17.09 | -79.7% |
| 8 | Water after Calcium Stearate removed | 20.61 | | |

**Table 5. Calcium Stearate Solution Tested after Suture removal**

| Solution | Endotoxin Detected (EU) |
|---|---|
| Water with 5% Calcium Stearate - sample 4 | <1.0 |
| Water with 5% Calcium Stearate - sample 5 | <1.0 |
| Water with 5% Calcium Stearate - sample 6 | <1.0 |

**Table 6. Calcium Stearate added**

| Sample | Mass of Calcium Stearate (grams) |
|---|---|
| 5% Calcium Stearate - sample 4 | 0.5139 |
| 5% Calcium Stearate - sample 5 | 0.5137 |
| 5% Calcium Stearate - sample 6 | 0.5181 |
| 5% Calcium Stearate Removed - sample 7 | 0.5120 |
| 5% Calcium Stearate Removed - sample 8 | 0.5221 |

Based on the above results, the treatment in an aqueous suspension of CaSt results in significantly decreased LPS or endotoxin activity, including on medical devices and in solutions contacted with medical devices.

### EXAMPLE 3. SUTURE TESTING IN ORGANIC SOLVENTS

A medical device (exemplified by an absorbable suture) was contaminated with LPS and then exposed to CaSt in a non-aqueous solvent and afterwards tested for LPS with a significantly decreased LPS concentration detected.

An absorbable VICRYL® (Polyglactin 910) suture (Size 1, Length: 27in) made of a copolymer made from 90% glycolide and 10% L-lactide was exposed to a solution containing LPS (endotoxin) (9 Strands of Vicryl suture were immersed in tubing with endotoxin solution (SRW, Concentration: 3980 EU/mL) for 10 minutes at a time at ambient temperature. The sutures were air dried for 20 minutes in a vented chemical hood and then placed in round bottom and dried The suture was then immersed into Ethyl Acetate (EtAc) containing CaSt, vortexed briefly for 10 seconds, dried for 20 minutes in a vented chemical hood, and extracted in endotoxin free water at ambient temperature. The suture extracted in endotoxin free water (LRW) was then tested for LPS using the Kinetic Chromogenic Method for Endotoxin Quantitation, Charles River Laboratories, Charleston S.C.

Table 7 shows the results of the experimental detection of LPS after exposure of the suture to CaSt in EtAc.

**Table 7. Detection of LPS after exposure of the suture to CaSt in EtAc**

| Sample | Solution Submerged In | Endotoxin Detected (EU) ) | Average Endotoxin Detected (EU) |
|---|---|---|---|
| 1 | Ethyl Acetate | 85.8 | 80.4 |
| 2 | Ethyl Acetate | 72 | |
| 3 | Ethyl Acetate | 83.4 | |
| | | | |
| 4 | Ethyl Acetate w/ 5% Calcium Stearate | 23.4 | 23.6 |
| 5 | Ethyl Acetate w/ 5% Calcium Stearate | 21.78 | |
| 6 | Ethyl Acetate w/ 5% Calcium Stearate | 25.62 | |
| | | | |
| 7 | No submersion in solvent | 107.52 | 105.6 |
| 8 | No submersion in solvent | 96.9 | |
| 9 | No submersion in solvent | 112.5 | |

Samples 1-3 were exposed to EtAc containing no CaSt as a control. As can be seen from the Table 7, about 80 EU of LPS was detected after the treatment in the solvent. Samples 4-6 were exposed to EtAc containing 5% Calcium Stearate. Referring again to Table 7, about 23 EU of LPS was detected after the treatment in the solvent. Samples 7-9 were not exposed to any solvent based treatment as a further control and about 105 EU of LPS was detected with no treatment in the solvent. CaSt in a solvent resulted in a significant reduction of LPS.

Similarly to the testing above, an absorbable suture of the same type, similarly contaminated, was exposed to CaSt in three solvents (ethanol, cyclohexane, isopropanol). The suture was then tested for LPS. Table 8 shows the results of the experimental detection of LPS after exposure of the suture to CaSt in these solvents. The solvents alone resulted in about 10% decrease in detected activity of LPS only. In presence of CaSt 1% suspension (CaSt could be slightly soluble in the solvents), LPS activity decreased by 75-95%.

Sutures used were 27 inch long pieces of absorbable lactide-glycolide sutures. Endotoxin sources used were Stagnant Rain Water (SRW) and LRW (3980 EU/mL). 7 strands of suture were placed in tubing with endotoxin solution for 10 minutes at a time. The sutures were dried for 20 minutes and then placed in round bottom tubes. Solutions of 1% by weight calcium stearate in different solvents (isopropanol, cyclohexane, and ethanol) were prepared. 1 suture was placed in each tube and vortexed in this solution for approximately 10 seconds. The sutures were removed and allowed to dry for 20 minutes and then placed in round bottom tubes. This was done for 6 sutures. 1 piece of suture, serving as the control, was not submerged in any solutions and placed in test tubes. 3 mL of LAL reagent water was added to each test tube, vortexed vigorously, and then placed in a hot water bath at 37°C for 1 hour. They were then removed, vortexed vigorously, diluted, and tested on the MCS system.

**Table 8. Results of testing in ethanol, cyclohexane, isopropanol**

| **Sample** | **Solution Submerged In** | **Endotoxin detected (EU)** | **Change in Endotoxin Level (%)** |
|---|---|---|---|
| Suture-1 | - | 68.1 | - |
| | | | |
| Suture-2 | Isopropanol | 60 | -11.9% |
| Suture-3 | Isopropanol w/1% Calcium Stearate (0.0841 g) | 16.59 | -75.6% |
| | | | |
| Suture-4 | Cyclohexane | 58.74 | -13.7% |
| Suture-5 | Cyclohexane w/1% Calcium Stearate (0.0858 g) | 4.02 | -94.1% |
| | | | |
| Suture-6 | Ethanol | 60.6 | -11.0% |
| Suture-7 | Ethanol w/1% Calcium Stearate (0.0827 g) | 15.06 | -77.9% |

Based on the above results, the treatment in a solvent with CaSt results in significantly decreased LPS or endotoxin activity detectable.

### EXAMPLE 4. WOUND DRESSING TESTING IN A SOLVENT

A wound dressing containing dry fibrinogen and dry thrombin on an absorbable support was tested as follows. The pad was spiked with LPS and then exposed to cyclohexane containing CaSt to determine the effect on detectable LPS.

Three 1x2 inch pieces of the dressing were spiked with endotoxin resulting in 8.9 EU of LPS. The pieces were allowed to dry for 20 minutes, then placed into 50 mL polypropylene conical tubes. 7.5 mL of LAL reagent water +2.5 mL 0.25M tris buffer was added to Tube 1. 10 mL of cyclohexane with 0.1 g of CaSt was added to Tube 2. 10 mL of cyclohexane was added to Tube 3. The tubes were closed and then placed on the bottle roller rotator machine for 15 minutes so that the tube could continuously roll and the dressing was constantly in contact with liquid.

Tube 1 solution was then diluted and tested for endotoxin on the MCS system. The fibrin pad pieces from Tube 2 and Tube 3 were removed and placed in new tubes and extracted in 7.5 mL of LAL reagent water + 2.5 mL 0.25M tris buffer. The solutions were then tested on the MCS system for endotoxin (LPS). The results are presented in Table 9.

**Table 9. Results of testing of dressing in cyclohexane containing CaSt**

| Fibrin Pad | Endotoxin detected on Pad (EU) | Percent Endotoxin detected as compared to Tube 1 |
|---|---|---|
| Tube 1 - No Cyclohexane | 8.9 | - |
| Tube 2 - Treated with Cyclohexane + Calcium Stearate | 1.8 | 20.2% |
| Tube 3 - Treated with Cyclohexane | 6.0 | 67.4% |

The results indicate that that LPS decreased from 9 EU to 6 EU after a treatment in pure cyclohexane, and decreased form 9 EU to 2 EU after treatment in cyclohexane + CaSt, indicating that CaSt had a significant effect on decreasing activity of LPS on a medical device.

### EXAMPLE 5. TESTING WITH CHELATING AGENTS AND SURFACTANTS

Further testing was performed with addition of EDTA and Polysorbate 20 reagents to verify permanent removal of LPS/endotoxin activity, by adding EDTA and Polysorbate 20 to the extraction solutions to test for potential recovery of endotoxin activity when introduced to these two substances.

The testing was performed in aqueous solutions with added EDTA and Polysorbate 20, with suspended CaSt present in some solutions in varying quantities. Calcium stearate was weighed and spiked with 5mL of an endotoxin solution. The tubes were vortexed for 1 minute and placed in a 37C bath for 1 hour. The tubes were removed from the bath and again vortexed for 1 minute. The solutions were diluted and tested for endotoxin using the Kinetic Chromogenic Method for Endotoxin Quantitation, Charles River Laboratories, Charleston S.C. To measure EDTA's effects, 0.155 g of the 0.1 M compound was added to the calcium stearate extraction solution. Polysorbate 20's effects were measured by adding 4 microL of the compound to the extraction solution with calcium stearate and EDTA. These samples were rigorously vortexed and tested using the Kinetic Chromogenic Method for Endotoxin Quantitation, Charles River Laboratories, Charleston S.C. Surprisingly, LPS/endotoxin activity was not recovered in presence of EDTA and Tween 20. The results are presented in Table 10.

**Table 10. Detection of LPS in water in presence of CaSt and EDTA and Tween 20**

| Sample | Weight of Calcium Stearate (g) | Endotoxin Spiked (EU) | Endotoxin detected (EU) | Percent detected Endotoxin |
|---|---|---|---|---|
| Water (control) | 0 | 8330 | 8700 | 104.4% |
| 0.1M EDTA and 0.05% Tween20 (control) | 0 | 8330 | 7450 | 89.4% |
| CaSt suspension | 0.007 | 8330 | 3995 | 48.0% |
| CaSt suspension with 0.1M EDTA and 0.05% Tween20 | 0.007 | 8330 | 5050 | 60.6% |
| CaSt suspension | 0.018 | 8330 | 1487.5 | 17.9% |
| CaSt suspension with 0.1M EDTA and 0.05% Tween20 | 0.018 | 8330 | 1545 | 18.5% |
| CaSt suspension | 0.028 | 8330 | 235 | 2.8% |
| CaSt suspension with 0.1M EDTA and 0.05% Tween20 | 0.028 | 8330 | 255 | 3.1% |

The results indicate that, even in presence of a chelating agent and surfactant, there was no significant reduction or weakening of LPS treatment by CaSt, especially at the higher levels of 0.028 grams and 0.018 grams CaSt per mL,

### EXAMPLE 6. TESTING OF VARIOUS SOURCES OF LPS

Laboratory grown endotoxin and natural endotoxin vary significantly. Therefore, for the methods described above, the activity of endotoxin was tested with various sources of endotoxin. Laboratory grown endotoxin is purified while natural endotoxin will be present with other substances such as proteins and phospholipids. This method of removing endotoxin activity with calcium stearate was shown to be effective in removing endotoxin activity from various sources of natural endotoxin.

Laboratory grown endotoxin is typically purified via extraction with phenol, dialyzed, treated with acetic acid/95% ethanol, treated with ribonuclease/deoxyribonuclease and then dialyzed again against water. The laboratory grown endotoxin used in this experiment was supplied by Charles River Laboratories.

Natural airborne endotoxin was captured using an electret filter. Electret filters are produced from dielectric polymer fibers that develop an electrical charge when air flows past them, capturing the endotoxin along with other particles. The endotoxin was extracted from the filter by placing the filter in endotoxin free water for an extended period of time.

Two other natural sources of endotoxin were obtained from water samples in the surrounding environment. One source was a pool of sitting rain water and other being a small puddle on the side of the Raritan River. The sitting rain water (SRW) was purified via chlorination and 0.2 µm filter. The Raritan River Water (RRW) was not altered in anyway. The same testing procedure was conducted for all sources of endotoxin. From the data below, it can be seen that very similar trends of removal of endotoxin activity with the present inventive methods can be seen for all sources.

The testing was performed using procedures similar to these described in Example 1. The results are shown in Table 11.

**Table 11. Detection of LPS after adding CaSt to test tubes containing water spiked with LPS**

| Endotoxin Type | Weight of Calcium Stearate (g) | Endotoxin Spiked (EU) | Endotoxin Detected (EU) | Percent of Endotoxin Detected |
|---|---|---|---|---|
| SRW | 0 | 11100 | 9200 | 82.9% |
| SRW | 0.01 | 11100 | 2780 | 25.0% |
| SRW | 0.25 | 11100 | 113 | 1.0% |
| Dust Filter Extraction Water | 0 | 9280 | 12350 | 133.1% |
| Dust Filter Extraction Water | 0.01 | 9280 | 3440 | 37.1% |
| Dust Filter Extraction Water | 0.25 | 9280 | 28.5 | 0.3% |
| RRW | 0 | 4340 | 4970 | 114.5% |
| RRW | 0.01 | 4340 | 960 | 22.1% |
| RRW | 0.1 | 4340 | 165 | 3.8% |
| RRW | 0.25 | 4340 | 70 | 1.6% |
| RRW | 0.35 | 4340 | 115 | 2.6% |
| Charles River Standard | 0 | 8330 | 8700 | 104.4% |
| Charles River Standard | 0.007 | 8330 | 3995 | 48.0% |
| Charles River Standard | 0.018 | 8330 | 1487.5 | 17.9% |
| Charles River Standard | 0.028 | 8330 | 235 | 2.8% |
| Charles River Standard | 0.05 | 8330 | 190.5 | 2.3% |
| Charles River Standard | 0.15 | 8330 | 86.5 | 1.0% |
| Charles River Standard | 0.25 | 8330 | 38.5 | 0.5% |

The data shows that as the mass of CaSt increased, the level of endotoxin detected or recovered decreased and approached 0%. At the levels of 0.1 grams of calcium stearate and higher, the percent of endotoxin recovered was under 5% for all sources of endotoxin (both laboratory standard and 2 natural sources). CaSt resulted in significant decrease of activity of all sources of LPS.

### EXAMPLE 7. OTHER REAGENTS

Other reagents including waxes, paraffins were tested for endotoxin removal and the results are shown in Table 12.

**Table 12. Testing wax and paraffin**

| Source of Wax | Mass of Wax Used (g) | Endotoxin Spiked (EU) | Endotoxin Detected (EU) | Endotoxin Percent Detected |
|---|---|---|---|---|
| Beeswax | 0.2654 | 7750 | 6450 | 83.2 |
| Beeswax | 0.5328 | 7750 | 6300 | 81.3 |
| "Burts Bee's" Lip Balm | 0.2559 | 7750 | 6800 | 87.7 |
| Paraffin Beads | 0.2516 | 7750 | 5580 | 72.0 |
| Paraffin Beads (Crushed) | 0.2535 | 7750 | 6800 | 87.7 |
| Paraffin Block | 0.2625 | 7750 | 4840 | 62.5 |
| Paraffin Candle Wax | 0.2526 | 7750 | 4585 | 59.2 |

As shown above, the inventors tested beeswax and found it did not work as effectively in decreasing LPS activity. Other salts of stearic acid were also tested and shown to have activity in decreasing LPS, albeit not as strong as CaSt. It is surprising that it was found that CaSt is significantly more effective than other reagents tested, such as waxes shown in Table 12.

### EXAMPLE 8. Using a mesh carrier coated with CaSt for LPS removal from water

### Experimental Procedure: Coating of polypropylene Mesh for endotoxin removal from water or solutions

Ethasew wax (Ethasew™ wax which is a mixture of 50 percent sorbitan monopalmitate, 20 percent sorbitan tri-stearate and 30 percent sorbitan tri-stearate containing 20 mole percent ethylene oxide) was mixed in a 50/50 weight ratio with Calcium stearate heated on a hot plate at 150°C mixed together and applied to a 2x1 inch piece of polypropylene mesh and let to cool.

Another mesh piece was prepared by mixing 10 ml of ethyl acetate in a weighing dish with 0.5g of Calcium Stearate. A 4x2 inch piece of polypropylene mesh was briefly submerged into the mixture of Ethyl Acetate and CaSt and removed to dry.

In several test tubes SRW and EVV samples containing endotoxin were contacted with the above treated mesh as well as tested as controls. EVV is a purified Endotoxin preparation in a suspension form, obtained from Charles River Laboratories. The tubes were then placed on a test tube roller for 15 minutes at 10 RPM, removed, diluted, and Endotoxin tested on the Charles River MCS. The results are shown in Table 13.

**Table 13. Testing of Mesh coated with CaSt**

| **Product** | **Endotoxin (EU/mL) detected** | **Percent Change (Endotoxin Removal)** |
|---|---|---|
| **SRW:** Spiking Solution Baseline | 127 EU/mL | -- |
| **EVV:** Spiking Solution Baseline | 346 EU/mL | -- |
| EVV Control | 315 EU/mL | -9% |
| EVV + CaSt coated Mesh | 73.9 EU/mL | -79% |
| EVV + Ethasew wax Mesh | 239 EU/mL | -31% |
| SRW Control | 105.5 EU/mL | -17% |
| SRW+CaSt coated Mesh | 85.6 EU/mL | -33% |
| SRW+Ethasew wax Mesh | 94.5 EU/mL | -26% |

As shown above a mesh carrier was effective in removal of endotoxin or LPS from water, with pure CaSt more effective, but with other forms of salts of stearic acid also somewhat effective in removal of endotoxin activity.

### EXAMPLE 9. Calcium Stearate (CaSt) Efficacy for sonication based agitation of solutions

10 ml of LRW water (LAL reagent water, contains no endotoxin) containing 0.04 g of CaSt suspension was sonicated using Misonix Probe Sonicator, Model: S-4000, Amplitude: 30, Time: 30 seconds and then the suspension was stored for 1 month at ambient temperature. Then 1 ml of the above suspension was added to 10 ml endotoxin containing water SRW and the resulting endotoxin concentrations in the combined solution measured after 15 min and 30 min, with the results presented in Table 14.

**Table 14. Effects of sonication on efficacy of CaSt, with sonication performed 1 month prior to testing of endotoxin removal**

| Solution Type | Initial endotoxin EU/mL | Endotoxin EU/mL (15 min after addition of CaSt suspension) | Endotoxin EU/mL (30 min after addition of CaSt suspension | Percent Endotoxin Removal(15 min/30 min) |
|---|---|---|---|---|
| TEST 1 | 732.5 | 372 | 384 | 49.2%/47.6% |
| TEST 2 | 732.5 | 362 | 278 | 50.6%/62.0% |

As shown in the above Table, even after 1 month of shelf life, the CaSt suspension was effective in removing endotoxin at levels of about 48%-about 62% after only 15 or 30 min of contact.

### EXAMPLE 10. Effect of storage on efficacy of CaSt suspensions

Efficacy of pre-mixed CaSt suspensions (sonicated and vortexed) was tested, comparing efficacy of immediately prepared suspensions with suspensions having shelf life or dwell time of 90 min, 5 days, and 1 month prior to contact with endotoxin containing solutions.

### Immediate Mixing testing:

SONICATION TREATMENT: Calcium Stearate (CaSt) + SRW Sonicated samples: test tubes containing 0.04g of CaSt and 0.02g of CaSt in 10mL LRW were sonicated for 30 seconds at a magnitude of 30. 10 mL SRW was then introduced to the sonicated CaSt mixture. It was then vortexed for 5 seconds to fully incorporate the sonicated CaSt. The mixture was then tested after 15 minutes of interaction to measure the percent decrease of endotoxin.

VORTEXING TREATMENT: Calcium Stearate (CaSt) + SRW Vortexed samples: test tubes containing 0.04g of CaSt and 0.02g of CaSt in 10mL LRW were vortexed. 10 mL SRW was then introduced to the vortexed CaSt mixture. It was then vortexed for 5 seconds to fully incorporate the sonicated CaSt. The mixture was then tested after 15 minutes of interaction to measure the percent decrease of endotoxin.

These mixtures were then tested on the Charles River MCS at a dilution of 1:2000 after 15 minutes of interaction.

Testing of suspensions having shelf life or dwell time of 90 min, 5 days, and 1 month prior to contact with endotoxin containing solutions.

Test samples were prepared as described above, sonicated and vortexed, but were subjected to shelf life or dwell time of 90 min, 5 days, and 1 month prior to contact with endotoxin containing solution (SRW). After dwell time exposure, similarly to the immediate mixing test above, 10 mL SRW was then introduced to the sonicated or vortexed CaSt mixture. It was then vortexed for 5 seconds to fully incorporate the sonicated CaSt. The mixture was then tested after 15 minutes of interaction to measure the percent decrease of endotoxin. These mixtures were then tested on the Charles River MCS at a dilution of 1:2000 after 15 minutes of interaction.

*The 1 month shelf life sample was only tested at 0.04g CaSt and measured the percent removal after 15 minutes of interaction as well as 30 minutes after interaction.

The results of the above testing are presented in Tables 15 and 16.

**Table 15. Effects of dwell time or aging on efficacy of 0.04g suspensions of CaSt prepared bv sonication or vortexing.**

| (0.04 g CaSt) Pre-Mixed Calcium Stearate: Shelf Life Efficacy | | | | |
|---|---|---|---|---|
| Sample | EU Before | EU After | Percent Endotoxin Removed | Average Percent Change |
| Sonicated | | | | |
| Immediate Mixing | | | | |
| Sonicated CaSt #1 | 36,100 | 5,320 | 85% | 85% |
| Sonicated CaSt #2 | 36,100 | 5,800 | 84% | |

| Mixing after 90 min dwell time | | | | |
|---|---|---|---|---|
| Aged Sonicated CaSt #1 | 36100 | 6168 | 83% | 81% |
| Aged Sonicated CaSt #2 | 36,100 | 8,000 | 78% | |

| Mixing after 5 Days dwell time | | | | |
|---|---|---|---|---|
| Aged Sonicated CaSt #3 | 37,800 | 5,040 | 87% | 85% |
| Aged Sonicated CaSt #4 | 37,800 | 6,560 | 83% | |

| Mixing after 1 Month dwell time | | | | |
|---|---|---|---|---|
| Aged Sonicated CaSt #1 | 14650 | 7440 | 49.2% | 50% |
| Aged Sonicated CaSt #2 | 14650 | 7240 | 50.6% | |

| Vortexed | | | | |
|---|---|---|---|---|
| Immediate Mixing | | | | |
| Aged Vortexed CaSt #1 | 36,100 | 19,840 | 45% | 44% |
| Aged Vortexed CaSt #2 | 36,100 | 20480 | 43% | |

| Mixing after 90 min dwell time | | | | |
|---|---|---|---|---|
| Aged Vortexed CaSt #1 | 36,100 | 17,120 | 53% | 54% |
| Aged Vortexed CaSt #2 | 36,100 | 16,460 | 54% | |

| Mixing after 5 Days dwell time | | | | |
|---|---|---|---|---|
| Aged Vortexed CaSt #3 | 37,800 | 9,080 | 76% | 75% |
| Aged Vortexed CaSt #4 | 37,800 | 9,760 | 74% | |

**Table 16. Effects of dwell time or aging on efficacy of 0.02g suspensions of CaSt prepared by sonication or vortexing.**

| Pre-Mixed Calcium Stearate: Shelf Life Efficacy (0.02 g CaSt) | | | | |
|---|---|---|---|---|
| Sample | EU Before | EU After | Percent Endotoxin Removed | Average Percent Change |
| Sonicated | | | | |
| Immediate Mixing | | | | |
| Sonicated CaSt #1 | 36,100 | 8,960 | 75% | 74% |
| Sonicated CaSt #2 | 36,100 | 10,240 | 72% | |

| 90 min | | | | |
|---|---|---|---|---|
| Aged Sonicated CaSt #1 | 36,100 | 15120 | 58% | 61% |
| Aged Sonicated CaSt #2 | 36,100 | 12,960 | 64% | |

| 5 Day | | | | |
|---|---|---|---|---|
| Aged Sonicated CaSt #3 | 37,800 | 9,200 | 76% | 76% |
| Aged Sonicated CaSt #4 | 37,800 | 9,200 | 76% | |

| Vortexed | | | | |
|---|---|---|---|---|
| Immediate Mixing | | | | |
| Aged Vortexed CaSt #1 | 36,100 | 29,200 | 20% | 32% |
| Aged Vortexed CaSt #2 | 36,100 | 20,480 | 43% | |

| 90 min | | | | |
|---|---|---|---|---|
| Aged Vortexed CaSt #1 | 36,100 | 22,400 | 38% | 43% |
| Aged Vortexed CaSt #2 | 36,100 | 19280 | 47% | |

| 5 Day | | | | |
|---|---|---|---|---|
| Aged Vortexed CaSt #3 | 37,800 | 15,120 | 60% | 64% |
| Aged Vortexed CaSt #4 | 37,800 | 12,160 | 68% | |

The data indicates that CaSt suspensions were efficacious even after significant storage times elapsed after preparation, with no substantial decrease in efficacy.

EXAMPLE 11. Kinetics of removal of LPS when using different agitation techniques in presence of different stearate salts CaSt, MgSt, AlSt.

Tables 17, 18, 19 show data on kinetics of removal of LPS when using different agitation techniques in presence of CaSt, MgSt, AlSt.

Experimental Procedure: 3 samples for each stearate type (calcium stearate, aluminum monostearate, and magnesium stearate) were weighed into test tubes. 5mL of SRW was added to each of the tubes. Each solution was tested for endotoxin removal after agitation based on with sonication, vortexing, or test tube rolling. Sonication samples were sonicated at each indicated time interval for 30 seconds at a magnitude of 30. Vortex samples were vortexed at each indicated time interval for 30 seconds at 2500 RPM. Rolling samples were constantly rolling at 10 rpm. The samples were tested on the MCS for endotoxin at 1 minute, 20 minutes, 40 minutes, 60 minutes, and 80 minutes.

**Table 17. CaSt: kinetics of LPS removal when using different agitation techniques**

| Calcium Stearate | | | | |
|---|---|---|---|---|
| | Time (Minutes) | Sample | Endotoxin Detected (EU) | Percent (%) Removed |
| | | SRW Baseline | 1,502 | |
| Sonication | 1 | Calcium Stearate | 270 | 82% |
| | 20 | Calcium Stearate | 130 | 91.3% |
| | 40 | Calcium Stearate | 87.5 | 94.2% |
| | 60 | Calcium Stearate | 56.9 | 96.2% |
| | 80 | Calcium Stearate | 16.4 | 98.9% |
| Vortexing | 1 | Calcium Stearate | 451 | 70% |
| | 20 | Calcium Stearate | 301 | 80% |
| | 40 | Calcium Stearate | 139 | 90.7% |
| | 60 | Calcium Stearate | 173 | 88.5% |
| | 80 | Calcium Stearate | 104 | 93.1% |
| Rolling | 1 | Calcium Stearate | 1,230 | 18.1% |
| | 20 | Calcium Stearate | 1,050 | 30.1% |
| | 40 | Calcium Stearate | 630 | 58.1% |
| | 60 | Calcium Stearate | 637 | 57.6% |
| | 80 | Calcium Stearate | 451 | 70% |

**Table 18. AlSt: kinetics of LPS removal when using different agitation techniques**

| Aluminum Monostearate | | | | |
|---|---|---|---|---|
| | Time (Minutes) | Sample | Endotoxin Detected | Percent (%) Removed |
| | | SRW Baseline | 1,502 | |
| Sonication | 1 | Aluminum Monostearate | 626 | 58.3% |
| | 20 | Aluminum Monostearate | 406 | 73% |
| | 40 | Aluminum Monostearate | 375 | 75% |
| | 60 | Aluminum Monostearate | 434 | 71.1% |
| | 80 | Aluminum Monostearate | 291 | 80.6 |
| Vortexing | 1 | Aluminum Monostearate | 817 | 45.6% |
| | 20 | Aluminum Monostearate | 849 | 43.5% |
| | 40 | Aluminum Monostearate | 786 | 47.7% |
| | 60 | Aluminum Monostearate | 801 | 46.7% |
| | 80 | Aluminum Monostearate | 757 | 49.6% |
| Rolling | 1 | Aluminum Monostearate | 221* | 85.3% |
| | 20 | Aluminum Monostearate | 1,260 | 16.1% |
| | 40 | Aluminum Monostearate | 1,240 | 17.4% |
| | 60 | Aluminum Monostearate | 672 | 55.3% |
| | 80 | Aluminum Monostearate | 1,220 | 18.8% |

**Table 19. MgSt: kinetics of LPS removal when using different agitation techniques**

| Magnesium Stearate | | | | |
|---|---|---|---|---|
| | Time (Minutes) | Sample | Endotoxin Detected (EU) | Percent (%) Removed |
| | | SRW Baseline | 1,502 | |
| Sonication | 1 | Magnesium Stearate | 743 | 50.5% |
| | 20 | Magnesium Stearate | 716 | 52.3% |
| | 40 | Magnesium Stearate | 824 | 45.1 |
| | 60 | Magnesium Stearate | 884 | 41.1% |
| | 80 | Magnesium Stearate | 703 | 53.2% |
| Vortexing | 1 | Magnesium Stearate | 884 | 41.1% |
| | 20 | Magnesium Stearate | 1,000 | 33.4% |
| | 40 | Magnesium Stearate | 901 | 40% |
| | 60 | Magnesium Stearate | 958 | 36.2% |
| | 80 | Magnesium Stearate | 1,060 | 29.4% |
| Rolling | 1 | Magnesium Stearate | 1,060 | 29.4% |
| | 20 | Magnesium Stearate | 1,120 | 25.4% |
| | 40 | Magnesium Stearate | 1,060 | 29.4% |
| | 60 | Magnesium Stearate | 1,120 | 25.4% |
| | 80 | Magnesium Stearate | 1,190 | 20.8% |

It can be seen that CaSt was more efficacious in removal of LPS, reaching much higher removal percentage and faster. It is surprising that the inventors found that the calcium stearate is significantly more effective than other salt forms. Sonication was most effective, followed by vortexing, followed by tube rolling.

### COMPARATIVE EXAMPLE 12. EFFECTS OF AGITATION IN ABSENCE OF SALTS OF STEARIC ACID

A control test evaluating effects of agitation on LPS was performed and the results are presented in Table 16.. The testing was a control test measuring the initial concentration of the SRW sample and effects of agitation in absence of any reagent added. 5 mL of SRW was added to 3 separate test tubes. "SRW Sonicated" was sonicated for 30 seconds at a magnitude of 30, Misonix Inc. Ultrasonic Liquid Processor Farmingdale NY. "SRW vortex" was vortexed by using Fisher Scientific Digital Vortex Mixer for 30 seconds at 2,500rpm, "SRW Roll" was rolled on a test tube roller, Thermo Scientific for 30 seconds at 10rpm. Extracts from these test samples were immediately diluted to 1:4000 and tested using the MCS. The test samples were diluted and tested again after 120 minutes to measure the endotoxin levels once more.

The results presented in Table 20 show minimal effects of agitation on the detected amounts of endotoxin in absence of any salt of stearic acid reagents.

**Table 20. Effects of agitation on LPS in absence of any reagents**

| **Treatment Controls** | | | |
|---|---|---|---|
| **Time (Minutes)** | **Sample** | **Endotoxin Detected (EU)** | **Percent (%) Removed** |
| | SRW Baseline | 1,502 | |
| 0 | Sonication Control | 1,236 | 17.7% |
| 120 | Sonication Control | 1,296 | 13.7% |
| 0 | Vortexing Control | 1,384 | 10.3% |
| 120 | Vortexing Control | 1,252 | 16.6% |
| 0 | Rolling Control | 1,304 | 13.2% |
| 120 | Rolling Control | 1,252 | 16.6% |

### EXAMPLE 13. Animal Testing.

An animal study was conducted with 30 female rats with weights ranging from 250-300g and acclimated for 5 days. All rats were dosed with intraperitoneal injections using 18 gauge needles. The rats were broken into 6 groups. A negative control (NC-1) dosed 5 rats with 5mL of normal saline solution. A negative control (NC-2) dosed 5 rats with 0.2g Calcium stearate in 5mL normal saline solution. A positive control (PC-1) dosed 5 rats with 2.5 million EU in a 5mL saline solution. A second positive control (PC-2) was conducted dosing 5 rats with 5 million endotoxin units in a 5mL normal saline solution. Two experimental test groups were conducted. A test group (T-1) of 5 rats was dosed with a mixture of 2.5 million EU and 0.2g calcium stearate in 5mL of normal saline. A second test group (T-2) of 5 rats was dosed with a mixture of 5 million EU and 0.2g calcium stearate in 5mL of normal saline.

All animals were observed for 4 days with the exception of the positive control specimens who met humane endpoint criteria after 4 hours and were subsequently removed from study. The results presented in Table 21 showed no toxicity or adverse effects in the calcium stearate test group samples (T-1 and T-2) whereas there was 100% severe toxicity in the positive control samples who were only dosed with endotoxin. This demonstrates the efficacy of calcium stearates endotoxin neutralizing capability. The negative control samples also had no toxicity.

**Table 21. Results of animal study with CaSt dosing**

| Treatment Group Name | Number of Animals | Treatment Condition (supplied as individual doses) | Active Component Targeted Dose | Results |
|---|---|---|---|---|
| Negative Control 1 (NC-1) | 5 | 5.0 mL of normal saline | - | No Toxicity Observed |
| Negative Control 2 (NC-2) | 5 | Calcium Stearate in normal saline brought up to 5.0 mL, vortex 30 seconds before dosing | 0.2 g Calcium Stearate | No Toxicity Observed |
| Positive Control 1 (PC-1) | 5 | 1X endotoxin brought up to 5.0 mL with normal saline solution, vortex 30 seconds before dosing | 2,500,000 EU dosed IP | Severe Toxicity Observed (humane endpoint reached) |
| Positive Control 2 (PC-2) | 5 | 2X endotoxin brought up to 5.0 mL with normal saline solution, vortex 30 seconds before dosing | 5,000,000 EU dosed IP | Severe Toxicity Observed (humane endpoint reached) |
| Test 1 (T-1) | 5 | 1X endotoxin + Calcium Stearate brought up to 5.0 mL with normal saline solution, vortex 30 seconds before dosing | 2,500,000 EU + 0.2g Calcium Stearate + normal saline dosed IP | No Toxicity Observed |
| Test 2 (T-2) | 5 | 2X endotoxin + Calcium Stearate brought up to 5.0 mL with normal saline solution, vortex 30 seconds before dosing | 5,000,000 EU + 0.2g Calcium Stearate + normal saline dosed IP | No Toxicity Observed |

## Claims

1. A method for treating solutions containing one or more endotoxins at detectable levels comprising:
a. adding a suspension of a salt of stearic acid to said solution to reduce detectable amounts of said endotoxins; and
b. optionally removing the salt of stearic acid.

2. A method according to claim 1 wherein the solution is aqueous.

3. A method according to claim 1 wherein the solution is water and the salt of stearic acid is substantially not water soluble.

4. A method according to claim 1 wherein the salt of stearic acid is selected from calcium stearate, magnesium stearate and aluminum stearate.

5. A method according to claim 1 wherein the salt of stearic acid is calcium stearate and the solution is water or saline.

6. A method for treating medical devices comprising: a) contacting the devices to a suspension of a salt of stearic acid in water or in non-aqueous solvents to reduce detectable amounts of endotoxins on said medical devices.

7. The method of claim 6, wherein said device is a suture.

8. The method of claim 6 wherein the device is contacted with water and the salt of stearic acid is calcium stearate.

9. A suspension of a salt of stearic acid for use in a method of reducing detectable amounts of endotoxins on a mammal, wherein said method comprises contacting the tissue surface of the mammal with a suspension of a salt of stearic acid.

10. A suspension of a salt of stearic acid for use according to claim 9 wherein the salt of stearic acid is calcium stearate.

11. A suspension of a salt of stearic acid for use according to claim 9 wherein the suspension of a salt of stearic acid is delivered in combination with antibiotic therapy.

12. A suspension of a salt of stearic acid for use according to claim 9 where the suspension of a salt of stearic acid is delivered into/ onto the gastrointestinal tract of a mammal.

13. A suspension of a salt of stearic acid for use according to claim 11 wherein the suspension of a salt of stearic acid is delivered in a quantity at least equal to the quantity of antibiotic.

14. A suspension of a salt of stearic acid for use according to claim 13 wherein the suspension of a salt of stearic acid contains calcium stearate suspended in a water/saline carrier.

15. A suspension of a salt of stearic acid for use according to claim 9 wherein the suspension of a salt of stearic acid containing calcium stearate suspended in a water/saline carrier is used as a lavage to remove endotoxin from injury sites prior to surgery or to reduce the severity and/or occurrence of sepsis.

## Patentansprüche

1. Verfahren zur Behandlung von Lösungen, die ein oder mehrere Endotoxine in nachweisbaren Konzentrationen enthalten, umfassend:
a. Hinzufügen einer Suspension eines Stearinsäuresalzes zu der Lösung zur Reduzierung nachweisbarer Mengen der Endotoxine; und
b. fakultativ Entfernen des Stearinsäuresalzes.

2. Verfahren nach Anspruch 1, wobei die Lösung wässrig ist.

3. Verfahren nach Anspruch 1, wobei die Lösung Wasser ist und das Stearinsäuresalz im Wesentlichen nicht wasserlöslich ist.

4. Verfahren nach Anspruch 1, wobei das Stearinsäuresalz unter Calciumstearat, Magnesiumstearat und Aluminiumstearat ausgewählt ist.

5. Verfahren nach Anspruch 1, wobei das Stearinsäuresalz Calciumstearat ist und die Lösung Wasser oder Kochsalzlösung ist.

6. Verfahren zur Behandlung medizinischer Vorrichtungen, umfassend: a) Inkontaktbringen der Vorrichtungen mit einer Suspension eines Stearinsäuresalzes in Wasser oder in nicht-wässrigen Lösungsmitteln zur Reduzierung nachweisbarer Mengen von Endotoxinen auf den medizinischen Vorrichtungen.

7. Verfahren nach Anspruch 6, wobei die Vorrichtung ein Nahtmaterial ist.

8. Verfahren nach Anspruch 6, wobei die Vorrichtung mit Wasser in Kontakt gebracht wird und das Stearinsäuresalz Calciumstearat ist.

9. Suspension eines Stearinsäuresalzes zur Verwendung in einem Verfahren zur Reduzierung nachweisbarer Mengen von Endotoxinen auf einem Säugetier, wobei das Verfahren das Inkontaktbringen der Gewebeoberfläche des Säugetiers mit einer Suspension eines Stearinsäuresalzes umfasst.

10. Suspension eines Stearinsäuresalzes zur Verwendung nach Anspruch 9, wobei das Stearinsäuresalz Calciumstearat ist.

11. Suspension eines Stearinsäuresalzes zur Verwendung nach Anspruch 9, wobei die Suspension eines Stearinsäuresalzes in Kombination mit einer Antibiotikatherapie verabreicht wird.

12. Suspension eines Stearinsäuresalzes zur Verwendung nach Anspruch 9, wobei die Suspension eines Stearinsäuresalzes in/auf den Magen-Darm-Trakt eines Säugetiers verabreicht wird.

13. Suspension eines Stearinsäuresalzes zur Verwendung nach Anspruch 11, wobei die Suspension eines Stearinsäuresalzes in einer Menge verabreicht wird, die der Menge des Antibiotikums mindestens gleicht.

14. Suspension eines Stearinsäuresalzes zur Verwendung nach Anspruch 13, wobei die Suspension eines Stearinsäuresalzes in einem Wasser/Kochsalzlösung-Träger suspendiertes Calciumstearat enthält.

15. Suspension eines Stearinsäuresalzes zur Verwendung nach Anspruch 9, wobei die Suspension eines Stearinsäuresalzes, die in einem Wasser/Kochsalzlösung-Träger suspendiertes Calciumstearat enthält, als Spülung zur Entfernung von Endotoxin von Verletzungsstellen vor einer Operation oder zur Reduzierung der Schwere und/oder des Auftretens von Sepsis verwendet wird.

## Revendications

1. Procédé pour le traitement de solutions contenant une ou plusieurs endotoxines à des niveaux détectables comprenant :
a. l'ajout d'une suspension d'un sel d'acide stéarique à ladite solution pour réduire les quantités détectables desdites endotoxines ; et
b. éventuellement l'élimination du sel d'acide stéarique.

2. Procédé selon la revendication 1, la solution étant aqueuse.

3. Procédé selon la revendication 1, la solution étant de l'eau et le sel d'acide stéarique n'étant sensiblement pas soluble dans l'eau.

4. Procédé selon la revendication 1, le sel d'acide stéarique étant choisi parmi le stéarate de calcium, le stéarate de magnésium et le stéarate d'aluminium.

5. Procédé selon la revendication 1, le sel d'acide stéarique étant le stéarate de calcium et la solution étant de l'eau ou une solution saline.

6. Procédé pour le traitement de dispositifs médicaux comprenant : a) la mise en contact des dispositifs avec une suspension d'un sel d'acide stéarique dans l'eau ou dans des solvants non aqueux pour réduire des quantités détectables d'endotoxines sur lesdits dispositifs médicaux.

7. Procédé selon la revendication 6, ledit dispositif étant une suture.

8. Procédé selon la revendication 6, le dispositif étant mis en contact avec de l'eau et le sel d'acide stéarique étant le stéarate de calcium.

9. Suspension d'un sel d'acide stéarique pour une utilisation dans un procédé de réduction de quantités détectables d'endotoxines sur un mammifère, ledit procédé comprenant la mise en contact de la surface du tissu du mammifère avec une suspension d'un sel d'acide stéarique.

10. Suspension d'un sel d'acide stéarique pour une utilisation selon la revendication 9, le sel d'acide stéarique étant le stéarate de calcium.

11. Suspension d'un sel d'acide stéarique pour une utilisation selon la revendication 9, la suspension d'un sel d'acide stéarique étant administrée en combinaison avec thérapie par un antibiotique.

12. Suspension d'un sel d'acide stéarique pour une utilisation selon la revendication 9 où la suspension d'un sel d'acide stéarique est administrée dans/sur le tube digestif d'un mammifère.

13. Suspension d'un sel d'acide stéarique pour une utilisation selon la revendication 11, la suspension d'un sel d'acide stéarique étant administrée en une quantité au moins égale à la quantité d'antibiotique.

14. Suspension d'un sel d'acide stéarique pour une utilisation selon la revendication 13, la suspension d'un sel d'acide stéarique contenant du stéarate de calcium en suspension dans un support d'eau/de solution saline.

15. Suspension d'un sel d'acide stéarique pour une utilisation selon la revendication 9, la suspension d'un sel d'acide stéarique contenant du stéarate de calcium en suspension dans un support d'eau/de solution saline étant utilisée comme un lavage pour éliminer une endotoxine de sites de blessures avant une chirurgie ou pour réduire la gravité et/ou l'occurrence d'une septicémie.
